# FASCICULE DE BREVET EUROPEEN

(11) **EP 0 693 949 B1**
(45) Date de publication et mention de la délivrance du brevet: **06.05.1998**
(21) Numéro de dépôt: 95906461.9
(22) Date de dépôt: 07.02.1995
(51) Int. Cl.: A61M 5/32, A61M 5/24

(54) **MODULE INJECTEUR POUR UNE SERINGUE**
INJEKTIONSMODUL FÜR EINE SPRITZE
INJECTOR MODULE FOR A SYRINGE

(30) Priorité: 14.02.1994 CH 429/94
(43) Date de publication de la demande: 31.01.1996
(73) Titulaire: SANOFI, 75008 Paris (FR)
(72) Inventeur: MEYER, Gabriel, CH-1195 Dully (CH)
(74) Mandataire: Nithardt, Roland
(86) Numéro de dépôt international: IB9500080
(87) Numéro de publication internationale: WO9521647

(56) Documents cités:
- EP-A- 0 281 421
- EP-A- 0 288 879
- EP-A- 0 331 452
- WO-A-93/00942
- DE-U- 8 802 080
- FR-A- 2 300 578
- US-A- 4 631 057
- US-A- 4 927 417
- US-A- 4 994 045

## Description

La présente invention concerne un module injecteur constituant un sous-ensemble d'une seringue, ce module comportant un corps cylindrique, une aiguille montée à une extrémité de ce corps et un manchon de protection de l'aiguille, cylindrique et coaxial audit corps, ce manchon étant mobile entre une position rétractée dégageant l'aiguille et une position avancée qui masque l'aiguille, ledit manchon étant constitué d'une pièce unique rigide agencée pour être montée par emboîtement et coulissement axial sur le corps du module, et ledit corps cylindrique du module comportant une jupe entourant un embout porte-aiguille, qui comporte au moins une languette souple agencée pour coopérer avec un bourrelet intérieur annulaire dudit manchon cylindrique rigide pour former un dispositif anti-retour lorsque le manchon est amené dans ladite position avancée, et au moins une butée d'arrêt également agencée pour coopérer avec ledit bourrelet intérieur annulaire dudit manchon pour délimiter la course de ce manchon lorsqu'il est dans sa position avancée.

Elle concerne également une seringue préremplie à un ou à deux compartiments contenant au moins une substance médicinale ou un composant d'une substance médicinale à reconstituer par mélange avec un autre composant, cette seringue ayant d'une part un module injecteur comportant un corps cylindrique, une aiguille montée à une extrémité de ce corps et un manchon de protection de l'aiguille, cylindrique et coaxial audit corps, ce manchon étant mobile entre une position rétractée dégageant l'aiguille et une position avancée qui masque l'aiguille, et d'autre part un module conteneur-propulseur comportant au moins un récipient définissant le ou les compartiments contenant ladite substance médicinale ou composant d'une substance médicinale, et au moins un obturateur associé audit récipient, ledit obturateur étant agencé pour coulisser à l'intérieur du récipient pour jouer le rôle de piston, et ledit récipient étant agencé pour coulisser à l'intérieur du corps pour jouer le rôle de tige de piston.

Les deux organismes officiels américains "Food and Drug Administration "(FDA) et "American Society of Hospital Pharmacist" (ASHP), qui contrôlent et fixent les normes à respecter en matière de substances médicinales et de moyens d'administration de ces substances aux patients, recommandent, sans toutefois imposer pour le moment, l'utilisation de systèmes d'injection permettant une rétraction de l'aiguille à l'intérieur du système après usage. La tendance de l'industrie pharmaceutique est orientée dans ce sens et la demande du milieu hospitalier, et en particulier du personnel soignant, va également dans ce sens. En effet, la crainte de certaines maladies virales pousse légitimement les médecins et les infirmières à s'entourer d'un maximum de précautions pour éviter une contamination accidentelle suite à une piqûre faite au moyen d'une aiguille de seringue usagée, ou similaire.

De très nombreux systèmes ont été imaginés pour tenter de préserver le personnel soignant contre des blessures accidentelles. En 1931 déjà, un tel système a fait l'objet d'un brevet américain délivré sous le N^{o} 1,921,034. Une seringue, montée à l'intérieur d'un réceptacle réalisé en deux éléments télescopiques, permet d'effectuer une injection sans que le soignant soit en contact, même accidentellement, avec l'aiguille. Par conséquent, le principe de l'aiguille escamotable est connu au moins depuis cette date.

Le brevet US N^{o} 2,571,653 délivré en 1951 décrit une seringue dont le corps est initialement engagé dans un manchon à l'intérieur duquel l'aiguille est masquée de manière à permettre une manipulation sans danger. Au moment de l'utilisation, le manchon peut être amené dans une position rétractée par rapport au corps de la seringue ce qui rend l'aiguille apparente et permet l'utilisation effective de la seringue.

Tous ces systèmes ont l'inconvénient de ne pas assurer une protection ferme, efficace et irréversible de l'aiguille après utilisation de la seringue. En effet, les gardes connues sont constituées essentiellement par un manchon qui entoure la seringue hypodermique, y compris l'aiguille pendant le stockage, cette aiguille devenant apparente au moment de l'utilisation. Rien n'est prévu pour assurer l'escamotage de cette aiguille après une injection faite à un patient, dans le but de protéger le personnel soignant.

D'autres réalisations dérivées de ces dispositifs ont fait l'objet des brevets américains US 4,738,663, US 4,631,057, US 4,573,976 et US 4,425,120, qui décrivent diverses gardes pour des seringues hypodermiques connues en soi.

Le système décrit en particulier dans le brevet US 5,061,251 tente de résoudre ce problème. A cet effet, ce système est pourvu d'un ressort associé à la tige de piston, ce ressort permettant, lorsqu'il est comprimé, de faire apparaître l'aiguille hors du manchon de protection. Pour que le système puisse fonctionner, il faut que la friction du piston à l'intérieur du corps de la seringue soit supérieure à la contrainte exercée par le ressort, pour éviter que la substance médicinale ne soit évacuée hors de la seringue avant l'injection. Ce n'est que lorsque le ressort arrive en butée que le piston peut commencer à se déplacer et à refouler la substance injectable vers l'aiguille. La contre-réaction exercée par le ressort est constante et tend à rétracter l'aiguille à l'intérieur du manchon. De ce fait, une aspiration d'une solution au moyen de cette seringue n'est pas possible. De même, l'opération appelée "test veine", qui consiste à aspirer du sang du patient juste avant l'injection pour vérifier si l'aiguille est effectivement plantée dans une veine, n'est que difficilement possible. D'une main, l'opérateur devrait comprimer le ressort et le maintenir en contraction et de l'autre, il devrait tirer sur la tige du piston pour effectuer une aspiration.

Le système décrit dans la demande de brevet français publiée sous le No. 2 617 718 tente d'apporter une solution pour résoudre le problème de la protection de l'aiguille avant et après l'injection dans le cas d'une seringue préremplie.

Au stockage, la stérilité de l'aiguille est assurée par la coopération d'une portion tronconique d'une gaine de protection avec une portion tronconique d'un manchon, sous l'effet de la pression d'un ressort pourtant supposé à l'état détendu. La moindre pression effectuée sur cette gaine de protection provoque l'éloignement des éléments tronconiques, ce qui met l'aiguille en communication avec l'extérieur de la seringue. La stérilité de l'aiguille est ainsi aisément rompue et aucun moyen n'est prévu pour empêcher ce type d'accident.

Pour effectuer l'injection, l'opérateur doit appuyer fortement l'extrémité de la gaine de protection contre la peau du patient.

Les forces à appliquer sont très importantes, à la fois pour permettre à l'aiguille de traverser la paroi d'extrémité de la gaine, de comprimer fortement le ressort, de déloger le manchon pour permettre son appui sur le piston et d'expulser la solution.

Toutes ces forces s'exercent directement sur la peau du patient avec toutes les conséquences que cela comporte, notamment la formation de marques, d'hématomes, de douleurs continues durant le processus d'injection, etc.. Dès que l'opérateur cesse d'exercer une pression constante et progressive sur le fond du conteneur de la seringue, l'injection s'interrompt et la gaine de protection recouvre l'aiguille en la délogeant de sa position d'injection dans la peau du patient. Le test veine est impossible à réaliser. Après injection complète ou interrompue accidentellement, l'aiguille n'est pas protégée de manière irréversible par l'action de détente du ressort connecté à la gaine de protection. En effet, la moindre pression sur cette gaine fait apparaître l'aiguille, de sorte que le système n'offre aucune protection sûre pour le personnel soignant. Le risque de contamination accidentelle est évident.

D'autres réalisations sont illustrées par les publications européennes EP 0 288 879 et EP 0 331 452 qui décrivent des seringues pourvues d'un corps et d'un manchon de protection de l'aiguille, coulissant pour pouvoir être amené dans une position avancée après utilisation de la seringue. Le corps de la seringue comporte des rampes rigides et le manchon de protection de l'aiguille, qui est en fait un manchon destiné à préserver l'utilisateur contre une piqûre accidentelle après usage de la seringue, est pourvu de languettes élastiques qui coopèrent avec les rampes pour constituer un mécanisme d'encliquetage assurant le verrouillage en position du manchon.

Ces systèmes à "rampes rigides" présentent de grandes difficultés d'assemblage du manchon de protection par l'avant du corps de seringue. La rampe rigide implique que l'assemblage se fasse en force par la déformation élastique du manchon pour passer par-dessus les rampes et les arrêts. Cette souplesse du manchon est incompatible avec la notion de blocage irréversible du manchon en position avancée. Si le manchon est souple pour faciliter l'assemblage, la fonction anti-retour n'est pas assurée. Si le manchon est trop rigide, ou lorsque les différences de diamètre entre le diamètre intérieur du manchon et le diamètre extérieur du corps sont telles qu'il existe un fort serrage entre ces deux pièces pour garantir l'effet anti-retour, la force nécessaire pour permettre au manchon de passer les rampes rigides du corps est importante, que ce soit d'ailleurs lors de l'assemblage ou lors de la mise en position avancée, ce qui n'est pas acceptable. Les moyens de blocage peuvent être endommagés lors de l'assemblage et/ou les forces à appliquer par l'opérateur sont trop élevées pour permettre un usage agréable.

En outre, rien n'est prévu pour supprimer les ailettes sur le corps et permettre de réaliser une seringue susceptible d'être fabriquée à l'état prérempli à une cadence élevée. En effet la présence d'ailettes empêche le groupage des seringues par paquets et le traitement simultané de toutes les unités d'un paquet, et impose par exemple le transport individuel des seringues d'un poste d'assemblage ou de remplissage à un autre poste de travail.

De plus rien n'est prévu pour permettre l'assemblage du manchon avec le corps sans risque d'endommager les moyens de blocage, ni pour supprimer les frottements excessifs inévitables au passage des rampes lors de la mise en position avancée du manchon de protection par l'opérateur. Par ailleurs rien n'est également prévu pour assurer le guidage du manchon et son maintien dans le prolongement du corps lorsqu'il est amené dans sa position avancée.

D'autre part, étant donné que la garde est construite pour être élastique au passage des rampes rigides, il n'est pas possible de prévoir un bourrelet annulaire intérieur rigide qui permet de réaliser un verrouillage irréversible en position avancée quel que soit le diamètre extérieur du corps de seringue. Une telle construction ne pourrait éventuellement fonctionner que pour des seringues de grands diamètres. Cet inconvénient est renforcé par le fait que la garde prévue pour être montée sur le corps de seringue se termine du côté proximal par un rétrécissement. Ce rétrécissement empêche la réalisation d'un bourrelet intérieur rigide comportant des angles vifs en raison de la technique de démoulage par contredépouille qui est habituellement utilisée.

Le brevet US-A-4,927,417 décrit une seringue d'injection comportant un manchon de protection pourvu, dans une de ses formes de réalisation, de languettes et de bourrelets qui coopèrent pour définir des positions de blocage de ce manchon respectivement en position rétractée et en position avancée. La géométrie des composants qui déterminent ces positions ne permet pas de garantir la sécurité de l'utilisateur en assurant le blocage en position avancée du manchon.

Le brevet US-A-4994045 qui correspond au préambule de la revendication 1 décrit une seringue d'injection comportant une jupe pourvue d'une languette qui coopère avec une fente dans un manchon de protection pour former un dispositif anti-retour. Le fonctionnement de ce dispositif est complexe nécessitant des mouements de translation et de rotation pour assurer le blocage.

Tous les systèmes connus présentent des limitations à l'utilisation et, par ailleurs, aucun ne permet l'emploi de seringues préremplies industriellement et stériles, fabriquées par des équipements standards à des cadences élevées.

La présente invention se propose de pallier l'ensemble des ces inconvénients en réalisant un système qui apporte une solution efficace aux questions de fabrication à haute cadence, ainsi qu'aux problèmes d'utilisation, notamment en répondant aux exigences les plus récentes de la pharmacopée en matière de stockage, transfert ou injection d'une solution médicinale stérile dans toutes sortes d'utilisations, en particulier l'injection directe à un patient au moyen d'une aiguille devant être neutralisée après usage.

Ce but est atteint par le module injecteur selon la revendication 1 par le fait que ladite butée d'arrêt est constituée par un bourrelet annulaire protubérant disposé à l'extrémité de ladite jupe annulaire, en ce que ladite butée d'arrêt s'étend sur au moins un secteur circulaire et comporte un épaulement à angle vif qui délimite ledit bourrelet annulaire protubérant par rapport à ladite jupe, et en ce que ledit manchon comporte un bourrelet annulaire intérieur délimité par des épaulements à angles vifs par rapport à la paroi intérieure dudit manchon, ce bourrelet coopérant avec la languette souple pour former le dispositif anti-retour et coopérant avec la butée d'arrêt pour délimiter la course du manchon.

De préférence lesdits angles vifs que forment ces épaulements avec la paroi intérieure du manchon sont supérieurs ou égaux à 90° et sont respectivement complémentaires à l'angle de l'extrémité de ladite languette souple et à l'angle vif qui délimite le bourrelet annulaire protubérant du manchon par rapport à ladite jupe.

Dans une variante de réalisation, la butée d'arrêt est constituée d'un bourrelet protubérant s'étendant sur deux secteurs diamétralement opposés, respectivement disposés entre les languettes souples de la jupe.

Dans toutes les formes de réalisation, la languette souple comporte avantageusement, à l'opposé de son extrémité inclinée, un rebord arrondi agencé pour coopérer avec l'épaulement du bourrelet intérieur au moment de la mise en place dans la position avancée.

Ledit manchon peut avoir des parois latérales rigides entièrement fermées ou qui présentent plusieurs ouvertures centrées sur un cercle périphérique audit manchon, ces ouvertures étant agencées pour permettre le passage de mâchoires d'une pince de serrage des languette de la jupe.

La présente invention sera mieux comprise en référence à la description de diverse formes de réalisation et aux dessins annexés dans lesquels :
la figure 1 représente une vue en perspective d'une seringue selon l'invention, prête à l'utilisation,
la figure 2 est une vue similaire à celle de la figure 1, après l'utilisation,
les figures 3 et 4 représentent des vues en coupe axiale d'une seringue du type à deux composants, respectivement prête à l'utilisation et après l'utilisation,

les figures 5, 6 et 7 représentent schématiquement une forme de réalisation d'un module injecteur pour une seringue selon l'invention, la figure 5 illustrant une phase de montage du manchon par l'arrière du corps du module, la figure 6 étant une vue en perspective de la jupe annulaire du corps et la figure 7 illustrant la position du manchon de protection de l'aiguille,
la figure 5A représente une vue de détail agrandie, représentant des languettes souples du corps et un bourrelet intérieur du manchon,
la figure 5B représente une vue similaire à celle de la figure 5A, mais correspondant à une variante de construction,
la figure 5C illustre le mode de fonctionnement des languettes souples et du bourrelet intérieur,
les figures 8, 9 et 10 représentent des vues d'une autre forme de réalisation d'un module injecteur pour une seringue selon l'invention, dans lequel le montage du manchon se fait par l'avant du corps, similaires à celles des figures 5, 6 et 7,
les figures 11, 12 et 13 représentent une autre forme de réalisation d'une seringue selon l'invention, dans laquelle le manchon est monté par l'arrière du corps,
les figures 14, 15, 16 et 17 illustrent une autre forme de réalisation de la seringue selon l'invention, dans laquelle le manchon est monté par l'avant du corps,
les figures 18, 19, 20 et 21 illustrent une construction de la seringue selon l'invention dans laquelle le montage du manchon s'effectue par l'avant du corps,
les figures 22, 23 et 24 illustrent une seringue de prise de sang dans laquelle le manchon est monté par l'avant, et
les figures 25 et 26 illustrent une seringue du type carpule dentaire, dans laquelle le manchon peut être adapté indifféremment par l'avant ou par l'arrière du corps.

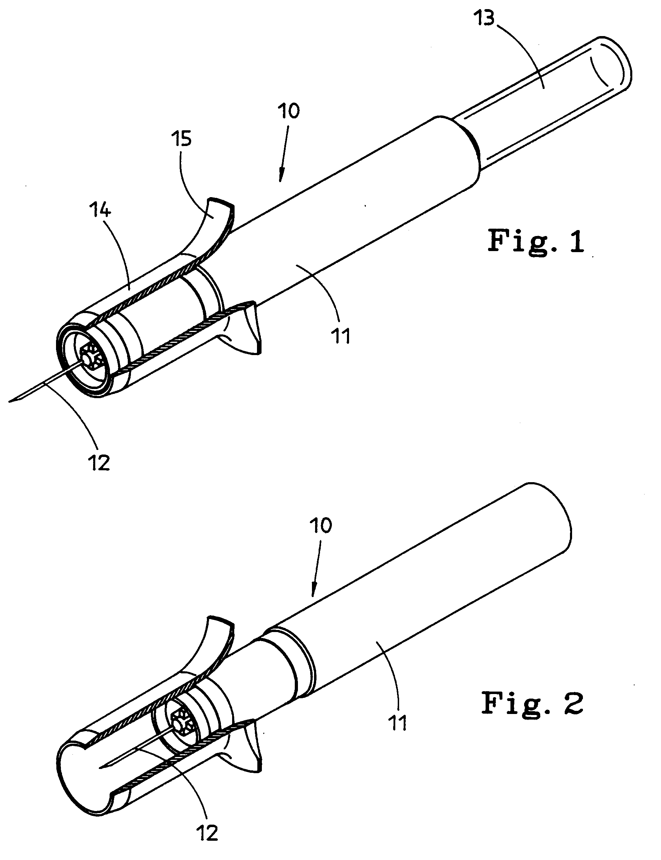

En référence aux figures 1 et 2, la seringue 10 comporte un corps cylindrique 11 équipé d'une aiguille 12 montée à une de ses extrémités et comportant un réservoir 13 contenant une substance médicinale. Le réservoir 13 peut comporter un seul compartiment si la seringue est du type à monocomposant ou deux compartiments, chacun de ces compartiments contenant alors un composant d'une substance médicinale à reconstituer. Le réservoir a une deuxième fonction qui est celle d'une tige de piston sur lequel l'opérateur appuie pour éjecter la substance médicinale en direction de l'aiguille 12 en vue d'une injection à un patient.

Par ailleurs, le corps de seringue est pourvu d'un manchon rigide de protection 14 de l'aiguille, cylindrique et coaxial à ce corps, et qui est mobile entre une position rétractée représentée par la figure 1 et une position avancée représentée par la figure 2. Ce manchon est pourvu d'éléments d'appui et de retenue 15 pour les doigts, qui peuvent se présenter sous la forme d'ailettes ou sous la forme d'une couronne circulaire réalisée d'une pièce avec le manchon. En fait, ce manchon équipé d'ailettes est une pièce en matière synthétique réalisée par moulage ou par injection et mise en place sur le corps de la seringue après toutes les autres opérations d'assemblage et de remplissage du réservoir. L'avantage de ce mode de réalisation est extrêmement important sur le plan de la fabrication industrielle de ce type de seringue préremplie. En effet, toutes les opérations d'assemblage et de montage, hormis la mise en place du manchon équipé d'ailettes, s'effectuent sur des pièces parfaitement cylindriques. De ce fait, toutes ces opérations peuvent être effectuées sur des éléments qui peuvent être serrés les uns contre les autres et positionnés de façon parfaitement ordrée. De plus, cet assemblage et ce remplissage peuvent être effectués sur des unités standards de très haute capacité et à des cadences extrêmement élevées étant donné que l'on peut supprimer toutes les phases transitoires où les composants passent de l'ordre au désordre et réciproquement. En conséquence, le fait de combiner les ailettes, qui sont nécessaires pour permettre une utilisation efficace et confortable de la seringue par le personnel soignant, avec le manchon destiné à protéger ce personnel contre des piqûres accidentelles au moyen de l'aiguille après utilisation de la seringue, c'est-à-dire lorsqu'elle est contaminée par le patient, permet non seulement de résoudre tous les problèmes de sécurité et de confort d'utilisation mais également d'améliorer considérablement la productivité des unités de fabrication.

Le corps 11, l'aiguille 12 et manchon de protection 14 de l'aiguille constituent le module injecteur qui est l'un des sous-ensembles de la seringue. Le réservoir 13 et un obturateur jouant le rôle de piston (non représenté sur les figures 1 et 2) constituent le module conteneur-propulseur qui est un autre sous-ensemble de la seringue.

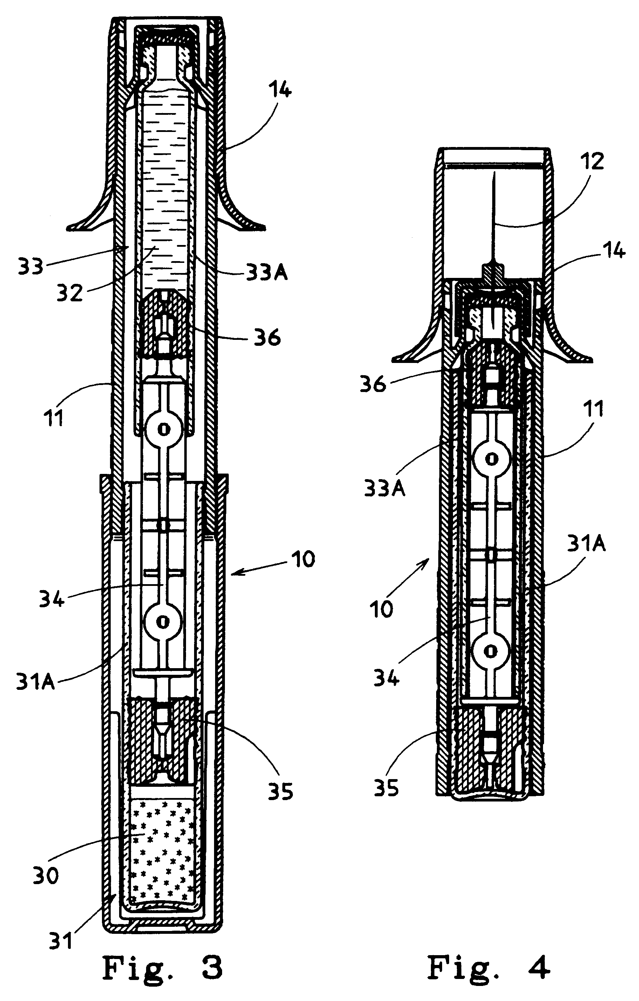

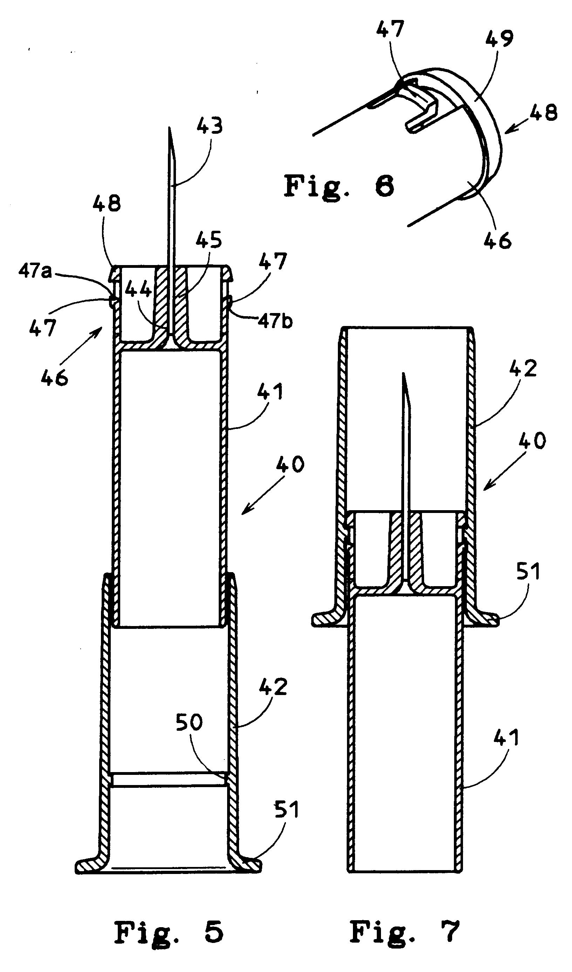

Les figures 3 et 4 représentent une forme de réalisation dans laquelle la seringue 10 est du type à deux composants, l'un des composants 30, par exemple sous forme d'une poudre ou d'un lyophilisat, étant contenu dans un compartiment 31 et un deuxième composant sous forme liquide 32 étant contenu dans un deuxième compartiment 33. L'ensemble des deux compartiments 31 et 33 constitue le réservoir de la substance médicinale injectable. Dans cette réalisation, le récipient est composé de deux réservoirs cylindriques 31A et 33A emboîtables télescopiquement, comme le montre plus particulièrement la figure 4. Ces deux réservoirs cylindriques 31A et 33A sont reliés par un tube de transfert 34 et montés au moins partiellement à l'intérieur du corps cylindrique 11 de la seringue. Le tube de transfert 34 assure, dans une première phase, le transfert du composant liquide 32 vers le compartiment 31 pour dissoudre le composant solide 30, et par la suite le transfert de la solution ainsi obtenue en direction de l'aiguille 12. Les deux compartiments sont respectivement obturés, pendant la phase de stockage par deux obturateurs-pistons-vannes respectivement 35 et 36. Pendant le stockage, ils jouent le rôle d'obturateurs. Dans une première étape de la phase d'utilisation, le mélange des deux composants s'effectue pour permettre la reconstitution de la substance médicinale qui constitue la solution injectable. Pendant l'étape suivante de la phase d'utilisation, ce mélange est injecté au patient. En fin d'utilisation, le manchon 14 est amené dans sa position avancée comme le montre la figure 4 et comme cela a été décrit précédemment. Dans cette position, il assure une protection efficace du personnel soignant contre une piqûre accidentelle. Comme dans la réalisation décrite précédemment, le corps 11, préalablement équipé de l'aiguille 12 fixée sur un embout porte-aiguille, et le manchon rigide de protection 14 constituent le module injecteur. Le module conteneur-propulseur est quant à lui composé des réservoirs cylindriques 31A et 33A, de la tige de transfert 34 et des deux obturateurs-pistons-vannes 35 et 36.

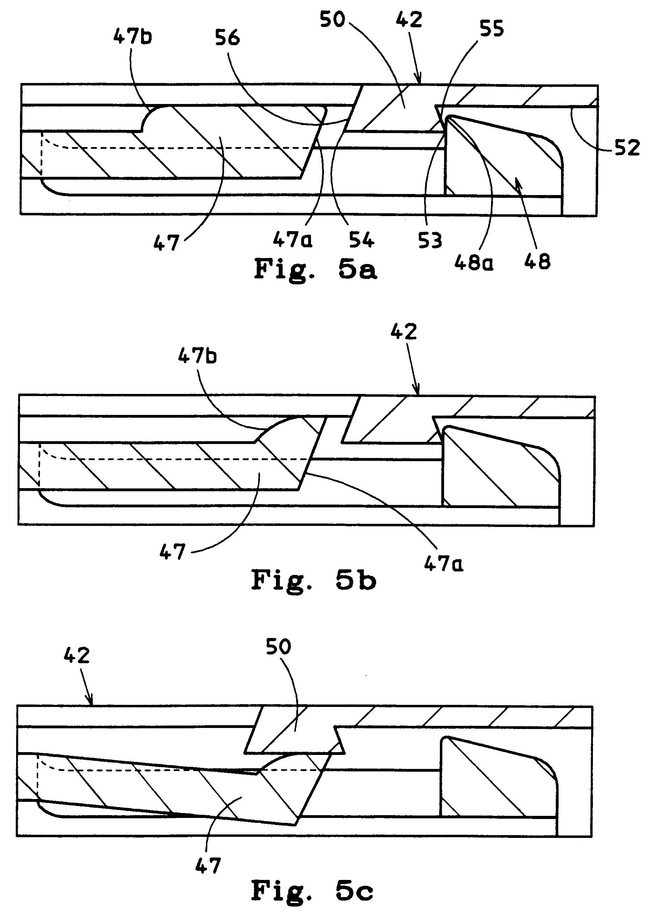

Les figures 5, 6 et 7 représentent schématiquement un module injecteur 40 pour une seringue comportant essentiellement un corps 41 de forme cylindrique et un manchon rigide 42 de protection de l'aiguille, cylindrique et coaxial au corps. Le manchon est prévu pour être monté sur le corps par l'arrière, c'est-à-dire l'extrémité opposée à l'extrémité qui porte l'aiguille 43, collée dans un canal approprié 44 ménagé à l'intérieur d'un embout porte-aiguille 45. Le module conteneur-propulseur n'est pas représenté. Il peut être conçu de différentes manières pour constituer soit une seringue du type jetable soit une seringue du type préremplie avec un réservoir indépendant jouant le rôle de tige de piston. Une des extrémités du corps du module est pourvue d'une jupe annulaire 46 qui comporte au moins une, mais de préférence deux ou plusieurs languettes souples 47 qui présentent une certaine flexibilité et qui, au repos, sont légèrement protubérantes vers l'extérieur. Par ailleurs, cette extrémité est équipée d'une butée d'arrêt 48 constituée par un bourrelet annulaire 49, protubérant vers l'extérieur et qui définit un rebord annulaire sensiblement perpendiculaire à ladite jupe annulaire 46.

Le corps du module, la jupe annulaire avec ses languettes souples et son bourrelet servant de butée d'arrêt, ainsi que l'embout porte-aiguille sont réalisés d'une pièce en matière synthétique injectée.

Le manchon 42 comporte un bourrelet intérieur 50 de forme annulaire et deux ailettes latérales ou, de préférence, un rebord annulaire 51 qui sert d'élément de retenue pour les doigts de l'utilisateur à l'injection.

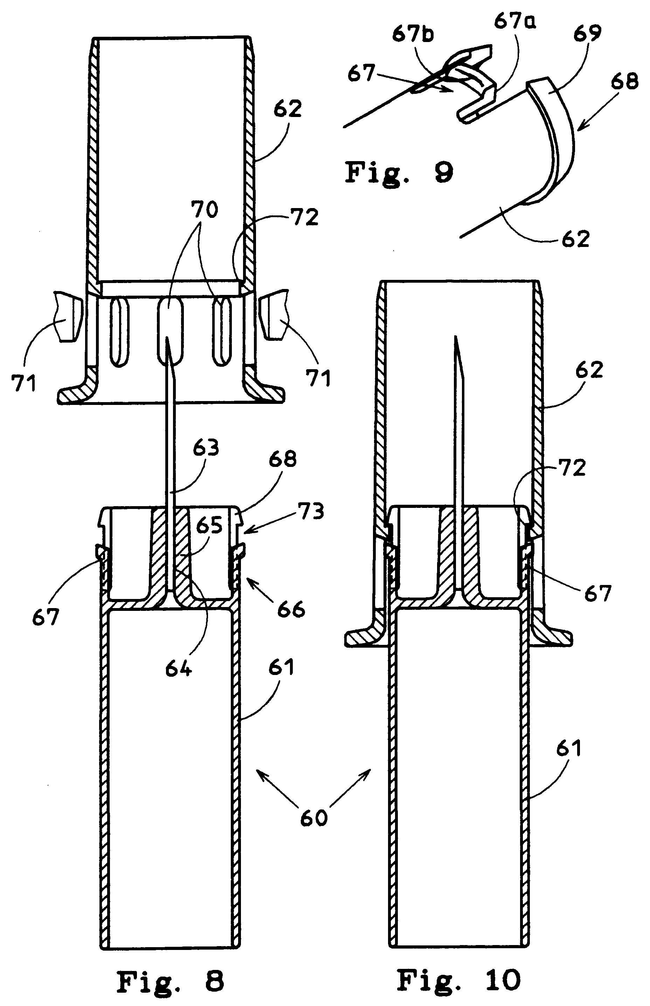

Ce manchon est conçu pour être mis en place par l'extrémité du corps du module opposée à l'extrémité portant l'aiguille 43. Son diamètre intérieur au niveau du bourrelet intérieur 50 est égal ou supérieur au diamètre extérieur du corps du module, de telle manière qu'il puisse coulisser sans difficulté vers l'extrémité portant ladite aiguille. Lorsque ce bourrelet, qui est rigide comme l'ensemble du manchon, arrive à la hauteur des ailettes, ces ailettes souples se rétractent vers l'intérieur de la jupe annulaire jusqu'à ce que le bourrelet soit en appui contre le rebord annulaire défini par le bourrelet annulaire 49 servant de butée d'arrêt. Comme la distance entre l'extrémité des languettes souples et ledit rebord annulaire est sensiblement égale ou légèrement supérieure à la largeur du bourrelet annulaire 50, les languettes souples se redressent après le passage du bourrelet annulaire 50, de sorte que le manchon 42 est immobilisé dans la position représentée par la figure 7. L'aiguille est parfaitement escamotée et le manchon protection de l'aiguille remplit sa fonction qui consiste à préserver le personnel soignant d'une piqûre accidentelle après l'utilisation de la seringue.

Comme le montre la vue agrandie 5A, le diamètre intérieur 52 du manchon 42 est approximativement le même de part et d'autre du bourrelet intérieur 50 de forme annulaire pour permettre le montage du manchon par l'avant ou par l'arrière. Ce bourrelet intérieur 50, qui n'est pas démoulé en force mais par l'emboîtement de deux moyeux métalliques disposés de part et d'autre de ce bourrelet, comporte des angles vifs 53 et 54. Ces angles vifs 53 et 54, qui sont inférieurs ou égaux à 90°, sont reliés au diamètre intérieur 52 du manchon 42 par des épaulements adéquats 55 et 56 inclinés pour permettre un accrochage parfait des languettes souples 47 et de la butée d'arrêt 48 lorsque le manchon 42 est en position avancée telle que représentée à la figure 7.

Pour coopérer avec l'épaulement 56, les languettes souples 47 présentent une extrémité 47a qui forme, avec l'axe longitudinal de la languette correspondante, un angle qui est sensiblement complémentaire auxdits angles vifs du bourrelet intérieur 50 du manchon 42 afin d'assurer un blocage anti-retour dudit manchon.

De façon similaire, la butée d'arrêt 48 peut comporter un angle vif 48a qui peut être compris entre 30 et 90° dans le but de coopérer avec l'épaulement incliné 55 du bourrelet intérieur 50.

A l'opposé de l'extrémité 47a de chaque languette, est prévu un rebord incliné, conique ou arrondi 47b qui, dans le présent cas, a un profil de quart de cercle ou similaire. Ce rebord arrondi est destiné à coopérer avec l'épaulement 55 du bourrelet intérieur 50, lorsque le manchon est amené vers sa position avancée. Grâce à ce rebord arrondi, le bourrelet intérieur repousse les languettes souples vers l'intérieur du corps de la seringue, ce qui permet la mise en place du manchon.

La figure 5B illustre une variante dans laquelle le profil du rebord 47b est un peu différent, en ce sens qu'il présente un rayon de courbure plus grand. En outre le tronçon d'extrémité de la languette 47, tronçon défini d'un côté par le rebord arrondi 47b de l'autre côté par l'extrémité 47a est plus court dans le cas de la figure 5B que dans le cas de la figure 5A.

La figure 5C illustre la coopération des languettes 47 avec le bourrelet intérieur 50. Au moment du passage du bourrelet, les languettes se rétractent élastiquement pour revenir dans leur position initiale et assurer le blocage en position du manchon 42.

Outre sa fonction de commande de la rétraction des languettes, le tronçon d'extrémité constitue un organe de guidage, puisqu'après le passage du bourrelet intérieur 50, les languettes ressortent élastiquement et prennent appui contre la paroi intérieure du manchon, assurant ainsi un guidage efficace de ce manchon.

L'emboîtement parfait du bourrelet intérieur 50 ayant des angles vifs 53 et 54, des languettes souples 47 et de la butée d'arrêt 48 ayant également des angles vifs complémentaires garantit le verrouillage irréversible de cet emboîtement en position avancée comme représenté à la figure 7.

Ces constructions spécifiques relatives à l'emboîtement peuvent être réalisées pour tous les manchons et pour les éléments des jupes annulaires représentés dans les autres figures.

Les figures 8, 9 et 10 illustrent un autre mode de réalisation d'un module injecteur 60 pour une seringue qui comporte comme précédemment un corps 61 de forme cylindrique et un manchon 62 de protection d'une aiguille 63 fixée par collage dans un canal 64 d'un embout porte-aiguille 65. L'extrémité du corps 61 portant l'aiguille d'injection est pourvue, comme dans la construction précédente, d'une jupe annulaire 66 équipée de languettes souples 67 et d'une butée d'arrêt 68 constituée par au moins une languette souple 69 portant le bourrelet annulaire. Cette languette souple 69 peut également être rigide.

Le manchon 62 est destiné à être monté par l'avant du corps 61, c'est-à-dire par l'extrémité du corps portant l'aiguille d'injection. Etant donné que les extrémités des languettes 67 sont protubérantes et empêchent en principe un tel montage, le manchon est pourvu d'ouvertures 70, centrées sur un cercle périphérique audit manchon, qui permettent l'introduction des mâchoires 71 d'une pince appropriée agencée pour resserrer momentanément lesdites languettes et autoriser le passage d'une bague rigide intérieure 72 par-dessus les languettes souples 67 et la butée d'arrêt 68, constituée par au moins une languette souple. Cette intervention n'est requise que pour la mise en place du manchon. En fin d'utilisation de la seringue, cette bague rigide 72, qui a la forme d'un bourrelet intérieur, se loge dans l'espace 73 qui sépare les languettes 67 de la butée d'arrêt 68, ce qui assure le blocage en position du manchon. Comme pour la réalisation selon les figures 5A, 5B et 5C les languettes souples 67 ont une extrémité 67a inclinée et un rebord 67b à profil arrondi, conique ou incliné.

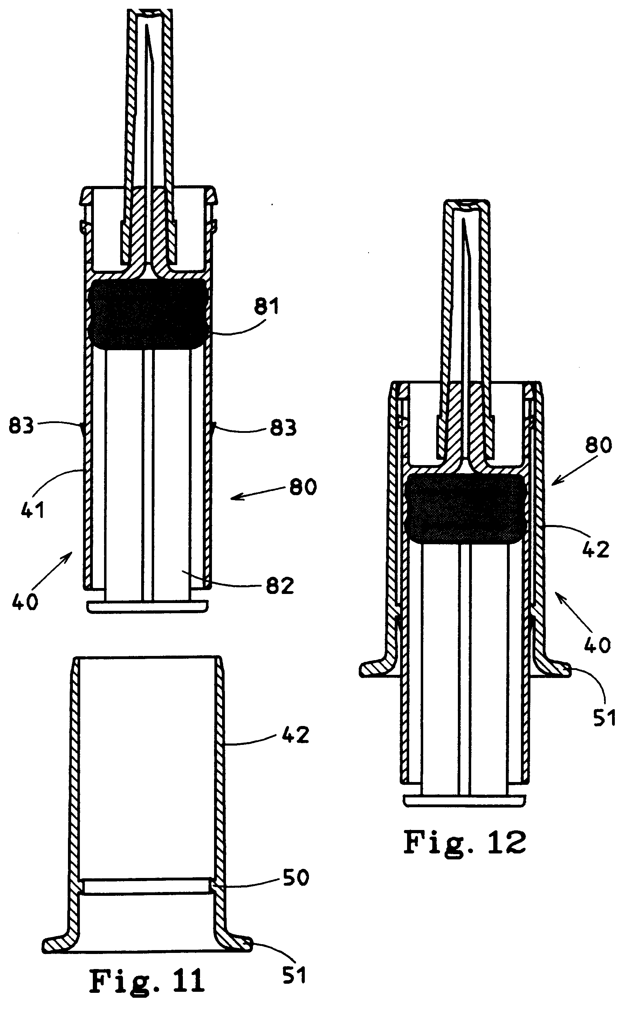

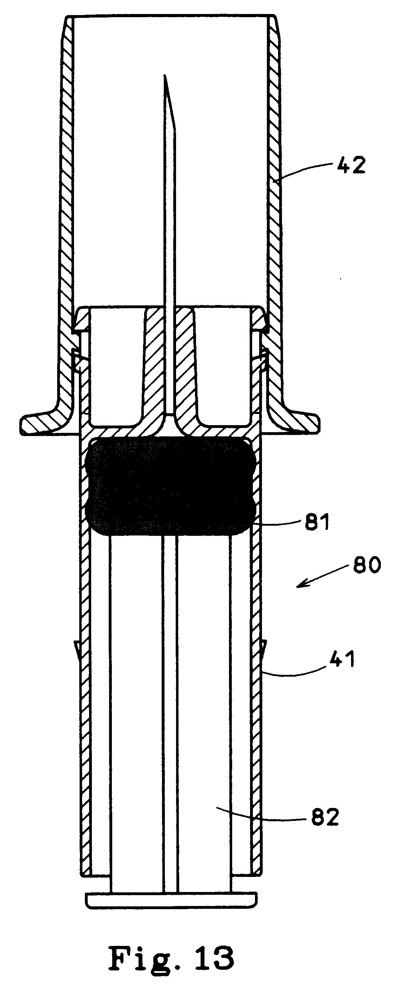

Les figures 11, 12 et 13 représentent une seringue jetable équipée d'un manchon monté par l'arrière tel que décrit en référence aux figures 5, 6 et 7. Dans ce cas, la seringue 80 se compose du module injecteur 40 et d'un module conteneur-propulseur formé de la partie intérieure du corps 41, d'un piston 81 et d'une tige de piston 82. Le manchon 42 est monté sur le corps 41 par l'extrémité dudit corps opposée à celle portant l'aiguille d'injection. Ce dernier est équipé d'au moins un, mais de préférence plusieurs ergots de blocage 83 qui coopèrent avec le bourrelet intérieur 50 pour assurer une fonction anti-recul du manchon. Cette fonction est indispensable étant donné que le manchon 42 porte les ailettes 51 qui permettent au praticien de tenir la seringue à l'aide de deux doigts et d'appuyer sur la tige du piston avec le pouce pendant l'injection. Cette position d'utilisation est illustrée par la figure 12. La figure 13 montre la seringue après l'injection et la mise en place du manchon dans sa position de sécurité.

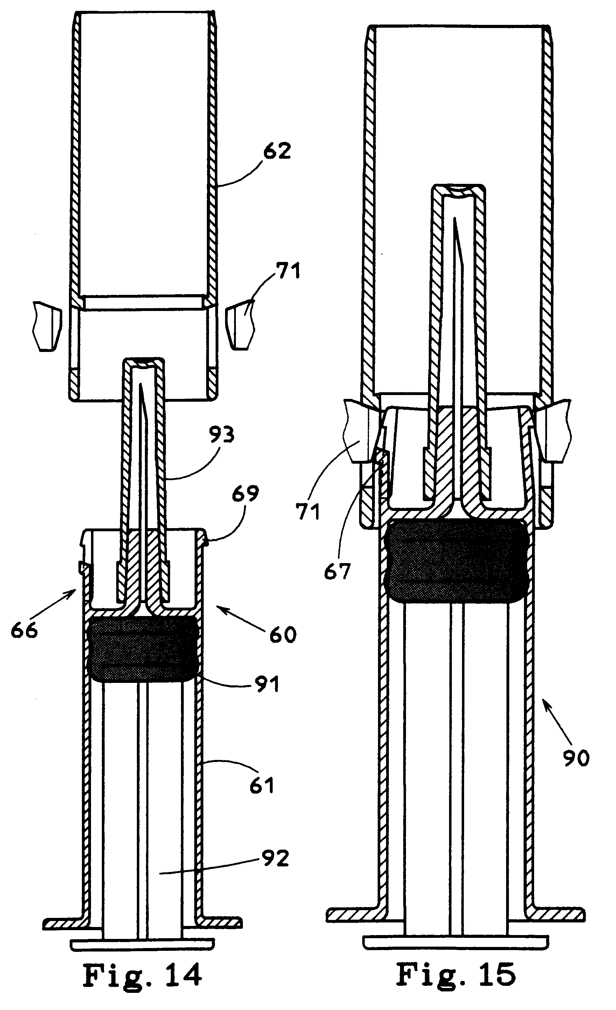

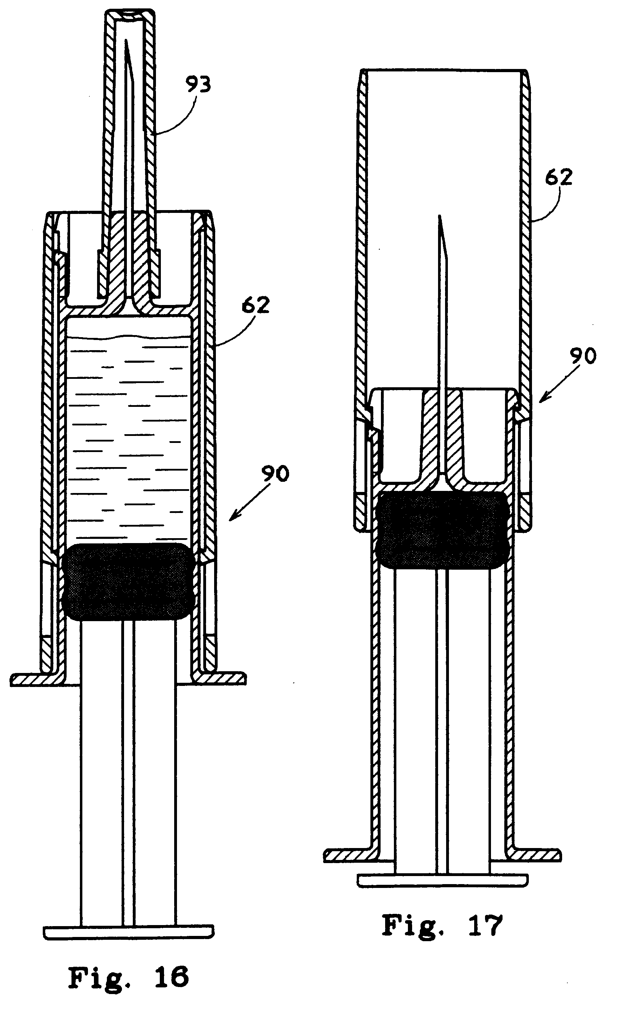

Les figures 14, 15, 16 et 17 représentent une seringue jetable équipée d'un manchon monté par l'avant, c'est-à-dire l'extrémité du corps portant l'aiguille d'injection, tel que décrit en référence aux figures 8, 9 et 10. La seringue 90 se compose du module injecteur 60, et d'un module conteneur-propulseur formé, comme dans l'exemple des figures 11, 12 et 13, de la partie intérieure du corps 61 et d'un piston 91 monté sur une tige de piston 92. En outre, dans ce cas l'aiguille est protégée par un capuchon 93. Il est bien évident qu'un tel capuchon pourrait également être adapté sur la seringue jetable 80 (voir figures 11, 12 et 13). La figure 14 montre la première phase de mise en place du manchon 62 sur le corps 61. La figure 15 montre une phase ultérieure au cours de laquelle les mâchoires 71 repoussent les languettes souples 67 de la jupe annulaire 66 et/ou les tronçons de jupe annulaire comportant le bourrelet annulaire 69, formant au moins une languette souple. La figure 16 montre la seringue qui sera prête à l'emploi dès que le capuchon 93 aura été retiré. La figure 17 montre cette seringue après utilisation, lorsque le manchon a été mis dans sa position de protection par l'utilisateur pour assurer sa protection.

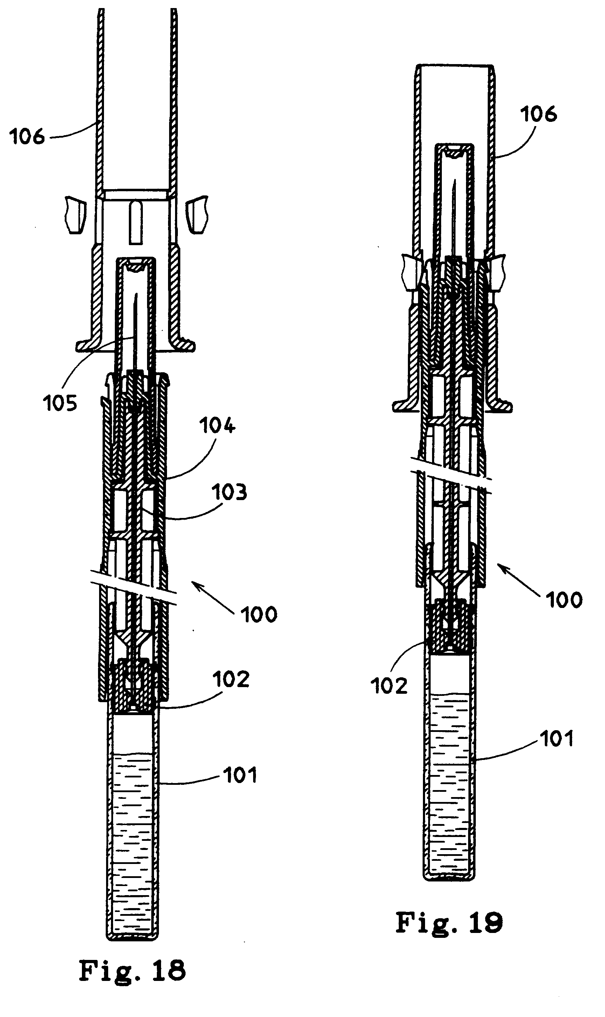

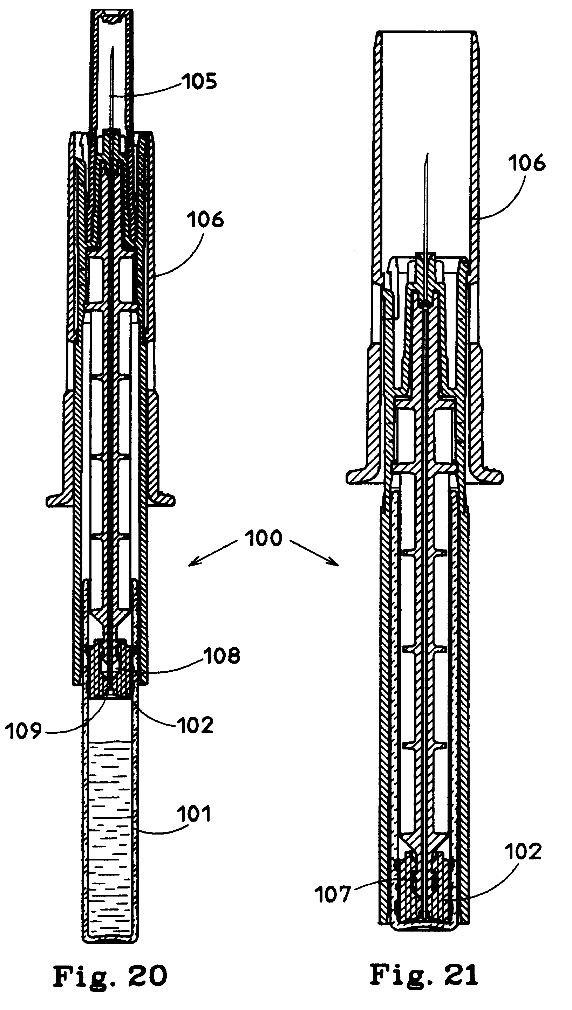

Les figures 18, 19, 20 et 21 illustrent une seringue 100 du type préremplie, monocomposant équipée d'un manchon monté par l'extrémité du corps portant l'aiguille d'injection. Cette seringue comporte un module injecteur similaire à celui décrit en référence aux figures 8 à 10. En revanche, le module conteneur-propulseur comporte dans ce cas un réservoir 101, de préférence en verre ou similaire, fermé à une extrémité et obturé à l'autre extrémité par un obturateur-piston-vanne 102, et un dispositif de transfert 103 monté dans le corps 104 du module injecteur. Dans une phase initiale, le dispositif de transfert, qui se compose essentiellement d'une tige tubulaire destinée à mettre en communication l'intérieur du réservoir 101 avec l'aiguille 105, n'est pas couplé à l'obturateur-piston-vanne 102. Cet organe joue le rôle d'obturateur. Après la mise en place du manchon 106 (voir figure 19), la seringue est prête pour le stockage. Cet état est représenté par la figure 20. La figure 21 montre la seringue en fin d'utilisation, lorsque le manchon a été amené dans sa position avancée. Entre ces deux phases, la préparation de la seringue à l'utilisation consiste à pousser le réservoir pour que le dispositif de transfert s'accouple à l'obturateur-piston-vanne 102. Cet accouplement s'effectue lorsqu'un embout 107, solidaire de l'extrémité correspondante du dispositif de transfert, s'emboîte dans la cavité centrale 108 de l'obturateur-piston-vanne 102 et que l'extrémité de la tige tubulaire de ce dispositif traverse une fente prédécoupée 109 dudit obturateur-piston-vanne. La pénétration de cette extrémité dans cette fente linéaire et ayant une hauteur relativement importante, permet de mettre l'aiguille 105 en communication directe avec l'intérieur du réservoir 101. On notera que l'épaisseur de la fente est comprise entre 1/5 et 1/3 de la hauteur de l'obturateur-piston-vanne 102 et que cette hauteur est supérieure au diamètre de l'extrémité de la tige tubulaire soit d'au moins 1 mm, de telle manière que la pression latérale sur ses lèvres assure une étanchéité absolue de l'obturateur pendant le stockage.

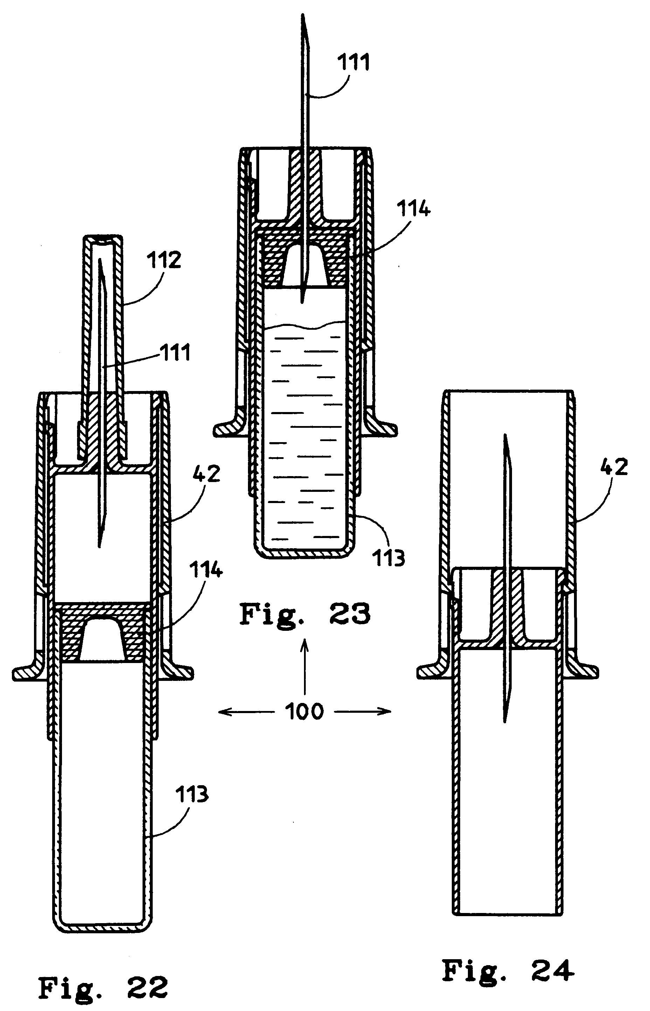

Les figures 22, 23 et 24 illustrent une seringue de prise de sang 110. Cette seringue comporte un module injecteur sensiblement identique à celui décrit en référence aux figures 5, 6 et 7. On notera toutefois que l'aiguille 111 est double et est protégée par un capuchon 112. Un flacon vide d'air 113, obturé par un bouchon 114, constitue dans ce cas le module conteneur-propulseur dont la fonction consiste à aspirer et contenir le sang d'un patient dans le cadre d'une opération de prise de sang classique, le bouchon 114 étant agencé pour être perforé par l'aiguille 111. La figure 22 montre la seringue avant l'usage, la figure 23 montre cette seringue en cours d'utilisation et la figure 24 la représente après usage, lorsque le manchon 42 a été amené dans sa position avancée assurant la protection du praticien et que le flacon rempli a été retiré.

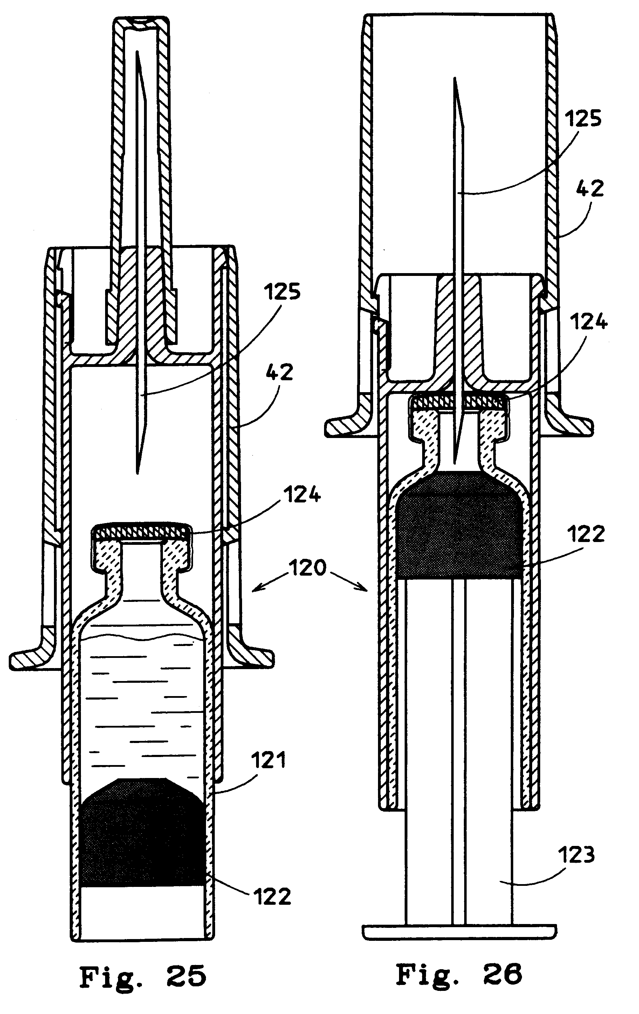

Les figures 25 et 26 illustrent une seringue spéciale 120 comportant une carpule dentaire 121. Le module injecteur est similaire à celui des figures 22, 23 et 24. Le flacon à vide d'air est remplacé par la carpule dentaire 121. Il s'agit d'un flacon obturé à une extrémité par une membrane et une capsule 124 agencées pour être perforées par une aiguille 125 du type double aiguille et dont le fond est obturé par un bouchon 122 qui assume la fonction d'obturateur au stockage (voir figure 25) et la fonction de piston lorsqu'il est associé à une tige de piston 123 (voir figure 26).

Comme dans les exemples précédents, en fin d'utilisation, le manchon 42 est amené dans sa position avancée qui permet d'assurer la protection du praticien contre une piqûre accidentelle.

Toutes ces réalisations démontrent que quel que soit son type de montage, le manchon peut être amené dans sa position avancée après l'utilisation de la seringue pour assurer une protection efficace du personnel soignant. En outre, comme il est équipé des ailettes de préhension de la seringue, les autres composants étant cylindriques sans présenter de grandes aspérités extérieures, les opérations d'assemblage, et le cas échéant de remplissage et de stérilisation, peuvent se faire en groupes serrés, le manchon à ailettes pouvant être rapporté, et la fabrication de ces produits est grandement facilitée et son coût de production fortement réduit.

## Revendications

1. Module injecteur constituant un sous-ensemble d'une seringue, ce module comportant un corps cylindrique, une aiguille montée à une extrémité de ce corps et un manchon de protection de l'aiguille, cylindrique et coaxial audit corps, ce manchon étant mobile entre une position rétractée dégageant l'aiguille et une position avancée qui masque l'aiguille, ledit manchon étant constitué d'une pièce unique rigide, agencée pour être montée par emboîtement et coulissement axial sur le corps (41) du module, et ledit corps cylindrique du module comportant une jupe (46) entourant un embout porte-aiguille (45) qui comporte au moins une languette souple (47, 67) agencée pour coopérer avec ledit manchon cylindrique rigide pour former un dispositif anti-retour lorsque le manchon est amené dans ladite position avancée, et au moins une butée d'arrêt (48) également agencée pour coopérer avec ledit manchon pour délimiter la course de ce manchon lorsqu'il est dans sa position avancée, caractérisé en ce que ladite butée d'arrêt (48) est constituée par un bourrelet annulaire protubérant (49) disposé à l'extrémité de ladite jupe annulaire (46), en ce que ladite butée d'arrêt (48) s'étend sur au moins un secteur circulaire et comporte un épaulement à angle vif qui délimite ledit bourrelet annulaire protubérant (49) par rapport à ladite jupe (46), et en ce que ledit manchon comporte un bourrelet annulaire intérieur (50) délimité par des épaulements (55, 56) à angles vifs (53, 54) par rapport à la paroi intérieure dudit manchon, ce bourrelet coopérant avec la languette souple pour former le dispositif anti-retour et coopérant avec la butée d'arrêt pour délimiter la course du manchon.

2. Module selon la revendication 1, caractérisé en ce que lesdits angles vifs (53, 54) que forment ces épaulements avec la paroi intérieure du manchon sont supérieurs ou égaux à 90°.

3. Module selon les revendications 1 et 2, caractérisé en ce que lesdits angles vifs (54, 53) sont respectivement complémentaires à l'angle de l'extrémité (47a) de ladite languette souple et à l'angle vif qui délimite le bourrelet annulaire protubérant (49) du manchon par rapport à ladite jupe.

4. Module selon la revendication 1, caractérisé en ce ladite butée d'arrêt est constituée d'un bourrelet protubérant (69) s'étendant sur deux secteurs circulaires diamétralement opposés, respectivement disposés entre les languettes souples (67) de la jupe (66).

5. Module selon les revendications 1 et 4, caractérisé en ce que ladite languette souple (47, 67) comporte à l'opposé de son extrémité inclinée (47a, 67a), un rebord arrondi (47b, 67b) agencé pour coopérer avec l'épaulement (55) du bourrelet intérieur (50), au moment de la mise en place du manchon (42, 62), dans sa position avancée.

6. Module selon la revendication 1, caractérisé en ce que ledit manchon (42) a des parois latérales rigides entièrement fermées.

7. Module selon les revendications 1 et 4, caractérisé en ce que ledit manchon (62) a des parois latérales rigides qui présentent plusieurs ouvertures (70) centrées sur un cercle périphérique audit manchon, ces ouvertures étant agencées pour permettre le passage de mâchoires (71) d'une pince de serrage des languette (67) de la jupe (66).

## Claims

1. An injector module forming of a syringe sub-assembly, this module comprising a cylindrical barrel, a needle extending from one extremity of this barrel and a sleeve protecting the needle, cylindrical and coaxial with said barrel, this sleeve being movable between a retracted position exposing the needle and an advanced position covering the needle, said sleeve consisting of a single rigid piece arranged to be telescopically interlocked with and axially slidable on the barrel (41) of the module, and said cylindrical barrel of the module comprising a skirt (46) surrounding a needle-holder tip (45) which comprises at least one flexible tab (47, 67) arranged to co-operate with said rigid cylindrical sleeve and to form an anti-return device when the sleeve is in the advanced position, and at least one stop means (48) also arranged to co-operate with said sleeve to define the path of this sleeve when it is in the advanced position, characterized in that said stop means (48) consists of a protruding annular rib (49) located at the end of said annular skirt (46), in that said stop means (48) extends along at least a circular portion and comprises an acutely angled side defining said protruding annular rib (49) in relation to said skirt (46), and in that said sleeve comprises an interior annular rib (50) defined by sides (55 56) forming acute angles (53, 54) in relation to the interior wall of said sleeve, this rib co-operating with the flexible tab to form the anti-return device and co-operating with the stop means to define the path of the sleeve.

2. A module according to claim 1, characterized in that said acute angles (53, 54) forming the sides with the interior sleeve wall are smaller than or equal to 90°.

3. A module according to claims 1 and 2, characterized in that said acute angles (54, 53) are respectively complementary to the angle of the extremity (47a) of said flexible tab and to the acute angle defining the protruding annular rib (49) on the sleeve in relation to said skirt.

4. A module according to claim 1, characterized in that said stop means consists of a protruding rib (69) extending across two diametrically opposed circular portions respectively located between said flexible tabs (67) of the skirt (66).

5. A module according to claims 1 and 4, characterized in that said flexible tab (47, 67) comprises, opposite its slanted extremity (47a, 67a), a curved edge (47b, 67b) arranged to co-operate with the side (55) of the interior rib (50) at the moment the sleeve (42, 62) is placed in its advanced position.

6. A module according to claim 1, characterized in that said sleeve (42) has rigid lateral walls which are completely closed.

7. A module according to claims 1 and 4, characterized in that said sleeve (62) has rigid lateral walls with comprise several openings (70) arranged in a peripheral circle on said sleeve, these openings being arranged to permit the passage of prongs (71) of a device for crimping the tabs (67) of the skirt (66).

## Patentansprüche

1. Injektionsmodul als Teilsystem einer Spritze, wobei das Modul ein zylindrisches Hauptteil, eine Nadel, die an einem Ende dieses Hauptteils befestigt ist, und eine zylindrische und koaxial zu dem Hauptteil liegende Schutzmuffe für die Nadel aufweist, wobei die Muffe zwischen einer zurückgeschobenen Stellung, welche die Nadel freigibt, und einer vorgeschobenen Stellung, welche die Nadel abdeckt, beweglich ist, wobei die Muffe aus einem einzigen starren Stück gebildet ist, welches so gestaltet ist, daß es axial auf das Hauptteil (41) des Moduls durch Aufstecken und Verschieben montierbar ist, und wobei das zylindrische Hauptteil des Moduls eine einen Nadelhalteransatz (45) umgebende Schürze (46) aufweist, welche mindestens eine flexible Zunge (47,67) aufweist, die so ausgeführt ist, daß sie mit der zylindrischen starren Muffe zusammenwirken kann, um eine Rückgangssicherung zu bilden, wenn die Muffe in die vorgeschobene Stellung gebracht ist, und mit mindestens einem Endanschlag (48), der ebenfalls so ausgeführt ist, daß er mit der Muffe zusammenwirken kann, um den Verschiebeweg der Muffe zu begrenzen, wenn sie in der vorgeschobenen Stellung ist, **dadurch gekennzeichnet,** daß der Endanschlag (48) als ringförmiger, vorspringender Wulst (49) ausgebildet ist, der am Ende der ringförmigen Schürze (46) vorgesehen ist, daß sich der Endanschlag (48) über mindestens einen kreisförmigen Sektor erstreckt und eine Schulter mit scharfer Kante aufweist, die den ringförmigen, vorspringenden Wulst (49) bezüglich der Schürze (46) begrenzt, und daß die Muffe einen inneren, ringförmigen Wulst (50) aufweist, der durch die Schultern (55,56) mit scharfen Kanten (53,54) bezüglich der Innenwand der Muffe begrenzt ist, wobei der Wulst mit der flexiblen Zunge zusammenwirkt, um die Rückgangssicherung zu bilden, und mit dem Endanschlag zusammenwirkt, um den Verschiebeweg der Muffe zu begrenzen.

2. Modul nach Anspruch 1,
**dadurch gekennzeichnet,** daß
die spitzen Winkel (53,54) welche die Schultern mit der Innenwand der Muffe bilden, kleiner oder gleich 90° sind.

3. Modul nach den Ansprüchen 1 und 2,
**dadurch gekennzeichnet,** daß
die spitzen Winkel (54,53) jeweils ergänzend zu dem Winkel des Endes (47a) der flexiblen Zunge und zu dem spitzen Winkel ausgeführt sind, der den ringförmigen, vorspringenden Wulst (49) der Muffe bezüglich der Schürze begrenzt.

4. Modul nach Anspruch 1,
**dadurch gekennzeichnet,** daß
der Endanschlag durch einen vorspringenden Wulst (69) gebildet ist, der sich über zwei kreisförmige, einander diametral gegenüberstehende Sektoren erstreckt, die jeweils zwischen den flexiblen Zungen (67) der Schürze (66) angeordnet sind.

5. Model nach den Ansprüchen 1 und 4,
**dadurch gekennzeichnet,** daß
die flexible Zunge (47,67) entgegengesetzt zu ihrem abgeschrägten Ende (47a,67a) eine abgerundete Kante (47b,67b) aufweist, die so ausgeführt ist, daß sie mit der Schulter (55) des inneren Wulstes zusammenwirken kann, und zwar im Augenblick der Befestigung der Muffe (42,62) in ihrer vorgeschobenen Stellung.

6. Modul nach Anspruch 1,
**dadurch gekennzeichnet,** daß
die Muffe (42) starre, vollkommen geschlossene Seitenwände hat.

7. Modul nach den Ansprüchen 1 und 4,
**dadurch gekennzeichnet,** daß
die Muffe (62) starre Seitenwände hat, die mehrere, auf einem peripheren Kreis der Muffe liegende, zum Zentrum hin gerichtete Öffnungen (70) aufweist, wobei diese Öffnungen so ausgeführt sind, daß sie die Backen (71) einer Zange zum Eindrücken der Zungen (67) der Schürze (66) durchlassen.
